# EUROPÄISCHE PATENTSCHRIFT

(11) **EP 2 034 879 B1**
(45) Veröffentlichungstag und Bekanntmachung des Hinweises auf die Patenterteilung: **12.08.2015**
(21) Anmeldenummer: 07764790.7
(22) Anmeldetag: 22.06.2007
(51) Int. Cl.: A61B 5/00

(54) **SYSTEM ZUR BESTIMMUNG DER POSITION EINES MEDIZINISCHEN INSTRUMENTES**
SYSTEM FOR DETERMINING THE POSITION OF A MEDICAL INSTRUMENT
SYSTÈME DE DÉTERMINATION DE LA POSITION D'UN INSTRUMENT MÉDICAL

(30) Priorität: 22.06.2006 DE 102006029122
(43) Veröffentlichungstag der Anmeldung: 18.03.2009
(73) Patentinhaber: amedo smart tracking solutions GmbH, 44799 Bochum (DE)
(72) Erfinder: TRÖSKEN, Volker, 58456 Witten (DE); HASENAU, Laszlo, 44789 Bochum (DE); GRÖNEMEYER, Dietrich, 58456 Witten (DE)
(74) Vertreter: Isfort, Olaf
(86) Internationale Anmeldenummer: PCT/EP2007/005520
(87) Internationale Veröffentlichungsnummer: WO 2007/147614

(56) Entgegenhaltungen:
- EP-A- 1 532 923
- WO-A-97/03609
- WO-A-02/096501
- WO-A-03/028547
- WO-A-2006/012491
- WO-A-2006/023674
- US-A1- 2003 060 702
- US-B1- 6 261 247
- US-B1- 6 490 473
- YIMIN ZHANG, MOENESS G. AMIN: 'Localization and Tracking of Passive RFID Tags' PROCEEDINGS OF SPIE Nr. 6248, 12 Mai 2006, Seiten 624809-1 - 624809-11 DOI: 10.1117/12.667773

## Beschreibung

Die Erfindung betrifft ein System zur Bestimmung der räumlichen Position und/oder Orientierung eines medizinischen Instrumentes, mit einer elektromagnetische Strahlung emittierenden Sendeeinheit, wenigstens einem an dem medizinischen Instrument angeordneten Lokalisierungselement, welches die von der Sendeeinheit emittierte elektromagnetische Strahlung empfängt und ein Lokalisierungssignal erzeugt, und mit einer Auswertungseinheit, welche die Position und/oder Orientierung des medizinischen Instrumentes durch Auswertung des Lokalisierungssignals bestimmt.

In der Medizin ist bei verschiedenen diagnostischen und therapeutischen Verfahren die genaue Bestimmung der Position eines verwendeten medizinischen Instrumentes von großer Bedeutung. Bei diesen Instrumenten kann es sich beispielsweise um intravaskuläre Katheter, Führungsdrähte, Biopsienadeln, minimal invasive chirurgische Instrumente oder ähnliches handeln. Von besonders großem Interesse sind Systeme zur Bestimmung der räumlichen Position und Lage eines medizinischen Instrumentes im Bereich der interventionellen Radiologie.

Ein System der eingangs angegebenen Art ist beispielsweise aus der EP 0 655 138 B1 bekannt. Bei dem vorbekannten System kommen mehrere Sendeeinheiten zum Einsatz, die an definierten Positionen im Raum verteilt angeordnet sind. Die Sendeeinheiten emittieren elektromagnetische Strahlung, ggf. mit jeweils unterschiedlicher Frequenz. Zur Lokalisierung des medizinischen Instrumentes ist an diesem ein Lokalisierungselement in Form eines Sensors angeordnet, welcher die von den Sendeeinheiten emittierte elektromagnetische Strahlung empfängt. Der Sensor detektiert das von den Sendeeinheiten erzeugten elektromagnetische Feld. Das von dem Sensor erzeugte Lokalisierungssignal entspricht der elektromagnetischen Feldstärke am Ort des Sensors und damit am Ort des medizinischen Instrumentes, an dem der Sensor angeordnet ist. Das Lokalisierungssignal wird einer Auswertungseinheit zugeführt. Die Auswertungseinheit berechnet aus dem Lokalisierungssignal den Abstand des Sensors zu den verschiedenen Sendeeinheiten. Da sich die Sendeeinheiten an definierten Positionen im Raum befinden, ist die Auswertungseinheit in der Lage, aus den Abständen des Lokalisierungelements von den verschiedenen Sendeeinheiten auf die Position des medizinischen Instrumentes im Raum zurückzuschließen.

Das vorbekannte System hat den Nachteil, dass das Lokalisierungselement über ein Kabel mit der Auswertungseinheit verbunden ist. Über das Kabel wird das von dem Lokalisierungselement empfangene Signal, das die Feldstärke der elektromagnetischen Strahlung am Ort des Lokalisierungselementes wiedergibt, der Auswertungseinheit zugeführt. Insbesondre zur Lokalisierung von medizinischen Instrumenten für minimal invasive Interventionen sind solche Kabelverbindungen höchst nachteilig. Die Ausstattung von minimal invasiven Instrumenten mit elektrischen Leitungen und zugehörigen Steckverbindungen ist aufwendig und damit teuer. Außerdem stören die elektrischen Zuleitungen bei der Handhabung der Instrumente.

Ein kabelloses System ist bekannt aus WO 2006/023674.

Vor diesem Hintergrund ist es Aufgabe der Erfindung, ein verbessertes System zu Bestimmung der räumlichen Position und/oder Orientierung eines medizinischen Instruments bereitzustellen.

Das Lokalisierungselement weist einen Transponder auf der eine Antenne und einen mit der Antenne verbundenen Schaltkreis zum Empfangen und Senden von elektromagnetischer Strahlung umfasst, wobei der Schaltkreis durch die über die Antenne empfangene elektromagnetische Strahlung der Sendeeinheit anregbar ist, und zwar in der Weise, dass dieser das Lokalisierungssignal als elektromagnetische Strahlung über die Antenne aussendet.

Vorteilhaft ist es, das medizinische Instrument mit einem Transponder auszustatten, wie er beispielsweise bei den bekannten RFID-Tags zum Einsatz kommt. Die Antenne des Transponders empfängt die von der Sendeeinheit emittierte elektromagnetische Strahlung und wird dadurch seinerseits zum Aussenden von elektromagnetischer Strahlung angeregt. Der Transponder sendet das Lokalisierungssignal somit ohne Kabelverbindung als elektromagnetische Strahlung aus. Die Auswertungseinheit ermittelt aus dem von dem Transponder abgestrahlten Lokalisierungssignal die räumliche Position und/oder Orientierung des medizinischen Instrumentes.

Verteilhaft ist, dass das Lokalisierungselement des Systems sehr kostengünstig herstellbar ist, da RFID-Tags Massenprodukte sind, die mit geringem Aufwand für die Verwendung angepasst werden können. Sehr kleine RFID-Transponder sind heute bereits kommerziell erhältlich. Die Antenne des Transponders kann zur Integration in ein medizinisches Instrument aus dünnem Draht als Spule gewickelt werden, wobei die Wicklungsrichtung und Geometrie der Spule an die Form und Größe des medizinischen Instrumentes beliebig angepasst werden kann.

Der Transponder des Lokalisierungselements funktioniert bei dem System wie die bekannten RFID-Transponder. Die Sendeeinheit erzeugt ein (hochfrequentes) elektromagnetisches Feld, welches die Antenne des Transponders empfängt. In der Antennenspule entsteht ein induktionsstrom. Dieser aktiviert den Schaltkreis des Transponders. Ist der Schaltkreis aktiviert, so sendet er seinerseits (hochfrequente) elektromagnetische Strahlung aus, beispielsweise in dem er das von der Sendeeinheit ausgesendete Feld (durch Lastmodulation) moduliert. Durch die Modulation liegt die von dem Transponder ausgesendete elektromagnetische Strahlung in einem Seitenband der Strahlung der Sendeeinheit. Auf diesem Seitenband wird das Lokalisierungssignal ohne Kabelverbindung, d. h. drahtlos, an die Auswertungseinheit zur Positionsbestimmung übertragen.

Der Transponder des Systems kann als passiver Transponder ausgebildet sein, wobei die Stromversorgung des Schaltkreises durch den beim Empfang der von der Sendeeinheit emittierten elektromagnetischen Strahlung in der Antenne erzeugten Induktionsstrom erfolgt. Diese Ausführungsform des Systems hat den Vorteil, dass der Transponder ohne eigene aktive Energieversorgung auskommt. Die Energie, die der Schaltkreis des Transponders zum Aussenden des Lokalisierungssignals benötigt, wird durch das von der Sendeeinheit erzeugte elektromagnetische Feld zur Verfügung gestellt. Zweckmäßigerweise weist der Transponder zur Stromversorgung des Schaltkreises einen Kondensator auf, der durch den in des Antenne erzeugten Induktionsstrom geladen wird. Der Kondensator sorgt für eine dauerhafte Energieversorgung des Schaltkreises. Zum Aufladen des Kondensators kann das medizinische Instrument in die Nähe der Sendeeinheit gebracht werden, wo das von der Sendeeinheit erzeugte elektromagnetische Feld ausreichend stark ist. Sobald der Kondensator geladen ist, funktioniert der Transponder für eine gewisse Zeit auch in größerem Abstand von der Sendeeinheit. Da die Energieversorgung durch den Kondensator gewährleistet ist, kann die Antenne des Transponders sehr klein dimensioniert werden, was die Integration in ein medizinisches Instrument erleichtert.

Alternativ kann der Transponder bei dem System als aktiver Transponder ausgebildet sein, wobei zur Stromversorgung des Schaltkreises eine Batterie vorgesehen ist. Die Aktivierung des Transponders erfolgt zweckmäßigerweise zu Beginn einer medizinischen Intervention, beispielsweise beim Öffnen der Verpackung des medizinischen Instrumentes. Alternativ ist der Schaltkreis des Transponders so ausgebildet, dass die Energieversorgung durch die Batterie erst bei Empfang der von der Sendeeinheit emittierten elektromagnetischen Strahlung aktiviert wird.

Gemäß einer sinnvollen Ausgestaltung des Systems unterscheidet sich die Frequenz der elektromagnetischen Strahlung des Lokalisierungssignals von der Frequenz der von der Sendeeinheit emittierten elektromagnetischen Strahlung. Dies ermöglicht es, das von dem Transponder ausgesendete Lokalisierungssignal von dem von der Sendeeinheit erzeugten elektromagnetischen Feld anhand der Frequenz zu unterscheiden. Realisiert werden kann dies, wie oben beschrieben, dadurch, dass der Transponder das Lokalisierungssignal durch Modulation der von der Sendeeinheit emittierten elektromagnetischen Strahlung erzeugt. Die Frequenz des Lokalisierungssignals liegt dann in einem Seitenband der Frequenz der von der Sendeeinheit emittierten elektromagnetischen Strahlung.

Gemäß einer vorteilhaften Weiterbildung des Systems ist die Auswertungseinheit mit wenigstens einer Empfangseinheit verbunden. Denkbar ist es, mehrere Empfangseinheiten zu verwenden, welche das von dem Transponder ausgesendete Lokalisierungssignal empfangen. Aus der Feldstärke des Lokalisierungssignals am Ort der jeweiligen Empfangseinheit kann auf dem Abstand des Transponders von der Empfangseinheit zurückgeschlossen werden. Wenn die Abstände des Transponders von den verschiedenen Empfangseinheiten, die sich an definierten Positionen im Raum befinden, bekannt sind, kann daraus mittels der Auswertungseinheit die genaue Position des Transponders und damit des medizinischen Instruments im Raum berechnet werden.

Problematisch ist allerdings, dass die Feldstärke des Lokalisierungssignals abgeschwächt wird, wenn das medizinische Instrument bei einer Intervention in den Körper eines Patienten eingeführt wird. Das Körpergewebe absorbiert aufgrund seiner dielektrischen Eigenschaften die von dem Transponder emittierte elektromagnetische Strahlung teilweise. Aus diesem Grund ist eine Positionsbestimmung anhand der Feldstärke des Lokalisierungssignals nicht immer mit ausreichender Genauigkeit möglich.

Zur Lösung dieses Problems kann die Auswertungseinheit zur Bestimmung der Position und/oder Orientierung des medizinischen Instrumentes anhand der Phasenlage der elektromagnetischen Strahlung des Lokalisierungssignals am jeweiligen Ort der Empfangseinheit eingerichtet sein. Bei geeigneter Wahl der Frequenz des Lokalisierungssignals ist der Einfluss der dielektrischen Eigenschaften des Körpergewebes auf die Phase des Lokalisierungssignals vernachlässigbar. Der Transponder sollte so eingerichtet sein, dass dieser das Lokalisierungssignal kohärent, d. h. mit definierter und konstanter Phasenlage emittiert.

Diese Technik ist bekannt aus beispielsweise "Localization and Tracking of Passive RFIDTags", Zhang, 2006.

Wenn die Positionsbestimmung, wie zuvor beschrieben, anhand der Phasenlage der elektromagnetischen Strahlung des Lokalisierungssignals erfolgt, ist zu beachten, dass eine eindeutige Zuordnung eines Phasenwertes zu einer Position im Raum nur innerhalb einer Distanz von dem Lokalisierungselement möglich ist, die kleiner ist als die Wellenlänge des Lokalisierungssignals. Bei größeren Distanzen ist es zusätzlich erforderlich, die Zahl der Nulldurchgänge der elektromagnetischen Strahlung des Lokalisierungssignals zwischen Lokälisierungselement und der jeweiligen Empfangseinheit zu bestimmen.

Aufgabe der Erfindung ist es, eine eindeutige Positionsbestimmung innerhalb eines möglichst großen Volumens mit hoher jenauigkeit durchzuführen.

Zur Lösung der Aufgabe der Erzielung einer möglichst hohen Genauigkeit bei der Positionsbestimmung wind fur den Transponder des Lokalisierungsetements bei dem erfindungsgemäßen System ein Schaltkreis verwendet, der zur Erzeugung des Lokalisierungssignals bei zwei oder mehr verschiedenen Frequenzen eingerichtet ist. Es kann durch die Erzeugung des Lokalisierungssignals bei niedrigen Frequenzen und entsprechend großen Wellenlängen zunächst eine grobe aber eindeutige Bestimmung der Position erfolgen. Zur Steigerung der Genauigkeit bei der Positionsbestimmung wird dann zu einer höheren Frequenz übergegangen, oder es wird die Frequenz des Lokalisierungssignals sukzessive weiter gesteigert. Bei höheren Frequenzen sind die Anforderungen an die Auflösung bei der Bestimmung der Phasenlage zur Erzielung einer bestimmten räumlichen Auflösung geringer. Bei der sukzessiven Steigerung der Frequenz kann die Zahl der Nulldurchgänge zur Bestimmung des genauen Abstands zwischen Lokalisierungselement und Empfangseinheit ermittelt werden. Zur möglichst genauen Positionsbestimmung ist eine Frequenzänderung in beiden Richtungen, d. h. von niedrigen zu hohen Frequenzen oder auch von hohen zu niedrigen Frequenzen, denkbar. In Abhängigkeit von den Frequenzbereichen, die zur Positionsbestimmung abgedeckt werden müssen, kann es erforderlich sein, zwei oder mehr Antennen vorzusehen, mit denen der Schaltkreis des Transponders verbunden ist, wobei jede der Antennen jeweils einem bestimmten Frequenzbereich zugeordnet ist.

Gemäß einer sinnvollen Weiterbildung des erfindungsgemäßen Systems ist der Transponder mit wenigstens einem Sensorelement verbunden, wobei der Schaltkreis des Transponders in der Weise eingerichtet ist, dass dieser das Sensorsignal des Sensorelements als elektromagnetische Strahlung über die Antenne des Transponders aussendet. Demgemäß wird der Transponder nicht nur zur Positionsbestimmung, sondern auch zur Übertragung von Sensorsignalen benutzt. Der Transponder ist mit entsprechenden Sensorelementen, beispielsweise einem Temperatursensor, einem Drucksensor, einem pH-Sensor oder auch mit einem herkömmliche Positionssensor, verbunden. Der Transponder überträgt das Sensorsignal drahtlos als Analog- oder Digitalsignal.

Die Leistungsfähigkeit des erfindungsgemäßen Systems kann weiter gesteigert werden durch wenigstens ein nicht an dem medizinischen instrument angeordnetes, zusätzliches Lokalisierungselement mit einem diesem zugeordneten Transponder, welches am Körper eines Patienten lösbar anbringbar ist. Das zusätzliche Lokalisierungselement kann beispielsweise mittels einer Klebe-, Adhäsion- oder Saugnapfverbindung lösbar an der Hautoberfläche des Patienten anbringbar sein. Gemäß einer besonders praktischen Ausgestaltung ist der Transponder des zusätzlichen Lokalisierungselements in einen selbstklebenden Folien- oder Gewebestreifen, wie bei einem herkömmlichen Pflaster, integriert. Mittels des zusätzlichen Lokalisierungselements kann die Position des Patienten bzw. eines bestimmten interessierenden Körperteiles des Patienten direkt zu der Position des medizinischen Instruments in Beziehung gesetzt werden. Dies ist insbesondere für Anwendungen der interventionellen Radiologie von Vorteil. Durch das zusätzliche Lokalisierungselement wird außerdem ermöglicht, Bewegungen des Körpers des Patienten bei der Positionierung des medizinischen Instruments zu berücksichtigen. So kann beispielsweise die Atembewegung des Patienten automatisch kompensiert werden, um die Genauigkeit der Nadelpositionierung bei Lungenbiopsien erheblich zu verbessern. Eine weitere Anwendung ergibt sich bei der Behandlung der Koronarien mit einem im Sinne der Erfindung ausgestalteten medizinischen Instrument (Katheter), um die atmungsbedingte Bewegung des Herzmuskels zu kompensieren. Ein weiterer Aspekt der Erfindung ist also die Verwendung eines RFID-Tags zur Integration in einen selbstklebenden Folie- oder Gewebestreifen zur lösbaren Anbringung an der Hautoberfläche eines Patienten.

Das erfindungsgemäße System kann mit Vorteil zur Positionsbestimmung bei MR-geführten operativen Eingriffen verwendet werden. Als Sendeeinheit des Systems kann sinnvollerweise die ohnehin vorhandene HochfrequenzSendeeinheit des MR-Gerätes genutzt werden. Diese umfasst eine Sende/Empfangsantenne, z.B. eine Körperspule in Form eines Käfig-Resonators, zur Erzeugung eines hochfrequenten elektromagnetischen Feldes im Untersuchungsvolumen des MR-Gerätes. Mittels eines solchen HF-Feldes werden bekanntlich kernmagnetische Resonanzen im Körper des untersuchten Patienten bei der MR-Bildgebung angeregt. Der Transponder kann in diesem Fall praktischerweise als passiver Transponder ausgebildet sein, wobei die Stromversorgung des Schaltkreises des Transponders durch den beim Empfang des HF-Feldes während der MR-Bildgebung in der Transponder-Antenne erzeugten Induktionsstrom, erfolgt. Zur Energieversorgung des Transponders wird demgemäß das im MR-Gerät ohnehin vorhandene HF-Feld ausgenutzt. Gemäß einer sinnvollen Weiterbildung des Systems kann die Auswertungseinheit mit dem MR-Gerät verbunden bzw. in dieses integriert sein, wobei die Bestimmung der Position und/oder Orientierung des medizinischen Instrumentes anhand des über die Sende-/Empfangsantenne des MR-Gerätes empfangenen Lokalisierungssignals erfolgt. Bei dieser Ausgestaltung wird also die Sende-/Empfangsantenne des MR-Gerätes zum Empfang des Lokalisierungssignals genutzt. Das Lokalisierungssignl wird über die Empfangselektronik des MR-Gerätes an die Auswertungseinheit übertragen. Besonders sinnvoll ist es, wie oben erläutert, die Position des Lokalisierungselernehtes anhand der Phasenlage des Lokalisierungssignals zu bestimmen. Entsprechend kann die mit dem MR-Gerät verbundene Auswertungseinheit mit Vorteil zur Bestimmung der Position und/oder Orientierung des medizinischen Instrumentes anhand der Phasenlage der elektromagnetischen Strahlung des Lokalisierungssignals am Ort der Sende-/Empfangsantenne des MR-Gerätes eingerichtet sein. Der Ort der Sende-/Empfangsantenne ist bekannt und unveränderlich. Von daher kann dieser Ort gut als Referenzpunkt bei der Positionsbestimmung anhand der Phasenlage herangezogen werden.

Bei medizintechnischen Systemen wird ein möglichst fehlersicherer (engl. "failsafe") Betrieb angestrebt. Das erfindungsgemäße System kann hierzu in der Weise ausgestaltet sein, dass die Auswertungseinheit (nach Art eines sogenannten "Voters") zur Auswahl gültiger Positions- und/oder Orientierungsdaten aus einer Mehrzahl von redundant aus mehreren Lokalisierungssignalen bestimmten Positions- und/oder Orientierungsdaten eingerichtet ist. Demgemäß werden aus den Lokalisierungssignalen zunächst redundante Positionsund/oder Orientierungsdaten bestimmt, beispielsweise indem in kurzem zeitlichem Abstand wiederholt Lokalisierungssignale aufgenommen werden oder indem Lokalisierungssignale von mehreren an dem medizinischen Instrument angeordneten Transpondern parallel aufgenommen werden. Diese redundanten Daten werden ausgewertet, miteinander verglichen und/oder auf Plausibilität überprüft. Nach dem Ergebnis dieser Überprüfung werden als gültig, d.h. als zutreffend erkannte Positions- und Orientierungsdaten ausgewählt. Es können beispielsweise diejenigen Positions- und Orientierungsdaten ausgewählt werden, die mit anderen redundant ermittelten Daten mehr oder weniger Übereinstimmung zeigen, während deutlich abweichende Daten (Ausreißer) als fehlerhaft erkannt und verworfen werden. Das Lokalisierungselement kann, wie oben erwähnt, eine Mehrzahl von Transponder aufweisen, die parallel und/oder zeitlich nacheinander zum Aussenden von Lokalisierungssignalen anregbar sind. Dies hat den Vorteil, dass ein fehlersicherer Betrieb selbst dann gewährleistet ist, wenn einzelne Transponder ausfallen oder deren Signale nicht oder (z.B. aufgrund von Störsignalen aus der Umgebung) verzerrt empfangen werden. Dies kann auch dadurch erreicht werden, dass an dem medizinische Instrument zur Erzeugung redundanter Lokalisierungssignale mehrere Lokalisierungselemente, jeweils mit einem oder mehreren Transponder, angeordnet sind. Redundanzen im Sinne einer hohen Fehlersicherheit können z.B. auch dadurch geschaffen werden, dass die Transponder zur Erzeugung der Lokalisierungssignale bei jeweils unterschiedlichen Frequenzen ausgelegt sind. Störungen in einzelnen Frequenzbereichen beeinträchtigen dann den sicheren Betrieb des Systems nicht.

Die Erfindung betrifft nicht nur ein System zur Positionsbestimmung, sondern auch ein medizinisches Instrument, das mit einem Transponder der zuvor beschriebenen Art ausgestattet ist, sowie ein Verfahren zur Bestimmung der räumlichen Position und/oder Orientierung eines medizinischen Instrumentes. Der Kerngedanke der Erfindung ist es, ein medizinisches Instrument, wie z. B. einen intravaskulären Katheter, einen Führungsdraht oder eine Biopsienadel, mit einem aktiven oder passiven RFID-Tag herkömmlicher Art auszustatten, um dadurch die Bestimmung der räumlichen Position und/oder Orientierung des medizinischen Instrumentes zu ermöglichen, vorzugsweise anhand der Phasenlage des von dem RFID-Tag erzeugten Lokalisierungssignals an einem festen Empfangsort. Außerdem kann das RFID-Tag genutzt werden, um, wie zuvor erläutert, Sensorsignale eines ebenfalls in das medizinische instrument integrierten Sensorelements drahtlos zu übertragen. Die Verwendung eines RFID-Tags in einem medizinischen Implantat ist ebenfalls denkbar, um Sensorsignale, wie z. B. Temperatur, Druck, pH-Wert oder auch Positionssignale, jederzeit vom Implantationsort abfragen zu können.

Sinnvollerweise kann der erfindungsgemäße Transponder einen Datenspeicher aufweisen, in welchem ldentifikationsdaten speicherbar sind, wobei der Schaltkreis zum Aussenden der ldentifkationsdaten als elektromagnetische Strahlung über die Antenne eingerichtet ist. Herkömmliche RFID-Tags weisen einen solchen digitalen Datenspeicher auf. Die ldentifikationsdaten können genutzt werden, um verschiedene Lokalisierungselemente bei der Bestimmung der räumlichen Position und/oder Orientierung voneinander zu unterscheiden. Wenn beispielsweise die Orientierung eines medizinischen Instrumentes, d. h. dessen Lage im Raum, bestimmt werden soll, ist es zweckmäßig, das Instrument mit wenigstens zwei erfindungsgemäß ausgebildeten Lokalisierungselementen auszustatten. Anhand der Positionen der beiden Lokalisierungselemente kann auf die Orientierung des Instrumentes zufückgäschlossen werden. Voraussetzung ist, dass eine Identifizierung der verschiedenen Lokalisierungselemente möglich ist, damit beispielsweise ein an der Spitze einer Biopsienadel angeordnetes Lokalisierungselement von einem an deren Griffteil angeordneten Lokalisierungselement unterschieden werden kann. Außerdem ist eine Identifizierbarkeit der Lokalisierungselemente sinnvoll, wenn mehrere medizinische Instrumente bei einer Intervention eingesetzt werden, da auf diese Weise gefährliche Verwechslungen bei der Positionsbestimmung vermieden werden.

Das erfindungsgemäße System kann, wie oben bereits erwähnt, zusammen mit einem bildgebenden diagnostischen Gerät, beispielsweise einem Computer-tomographen oder einem MR-Gerät, verwendet werden, um die Navigation mit dem verwendeten interventionellen Instrument zu ermöglichen. Die mittels des erfindungsgemäßen Systems bestimmten Positions- und Orientierungsdaten können gemeinsam mit den abgebildeten anatomischen Strukturen visualisiert werden, um dem die Intervention durchführenden Arzt die Führung des Instrumentes zu erleichtern. Dabei ist vorteilhaft, dass mit dem erfindungsgemäßen System die Bestimmung der räumlichen Position und/oder Orientierung unabhängig von dem Bildgebungssystem möglich ist. Somit kann die Strahlenbelastung bei minimal invasiven Interventionen reduziert werden. Die exakte Positionierung und Navigation der Instrumente ist nämlich ohne kontinuierliche Röntgendurchleuchtung möglich.

Ausführungsbeispiele der Erfindung werden im Folgenden anhand der Zeichnungen erläutert. Es zeigen:
- Fig. 1: Darstellung des erfindungsgemäßen Systems als Blockdiagramm;
- Fig. 2: schematische Darstellung eines erfindungsgemäß ausgestalteten medizinischen Instruments.

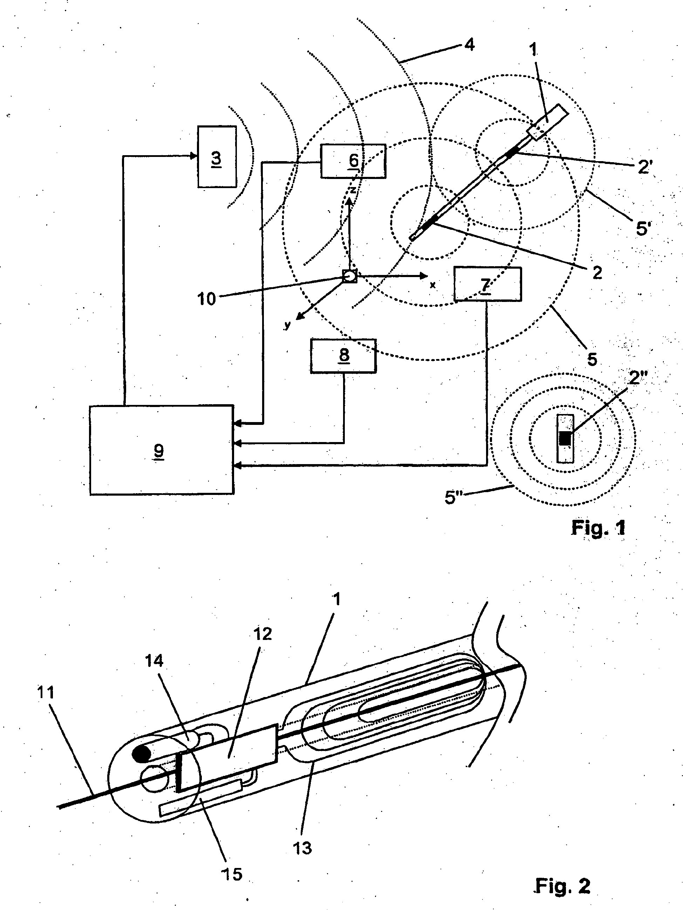

Das in der Fig. 1 dargestellte System dient zur Bestimmung der räumlichen Position und Orientierung eines medizinischen Instrumentes 1. An dem medizinischen Instrument 1 sind Lokalisierungselemente 2 und 2' angeordnet. Das System umfasst eine Sendeeinheit 3, die elektromagnetische Strahlung 4 emittiert. Die Strahlung 4 wird von den Lokalisierungselementen 2 und 2' empfangen. Die Lokalisierungselemente 2 und 2' weisen jeweils einen Transponder auf, welcher durch die empfangene Strahlung 4 angeregt wird, so dass der Transponder seinerseits das Lokalisierungssignal als (hochfrequente) elektromagnetische Strahlung 5 bzw. 5' aussendet. Die von den Lokalisierungselementen 2 und 2' abgestrahlten Lokalisierungssignale 5 und 5' werden von drei an definierten Positionen im Raum befindlichen Empfangseinheiten 6, 7 und 8 empfangen. Die Empfangseinheiten 6, 7 und 8 sind mit einer Auswertungseinheit 9 verbunden, welche anhand der Phasenlage der elektromagnetischen Strahlung 5 bzw. 5' der Lokalisierungssignale am jeweiligen Ort der Empfangseinheiten 6, 7 und 8 die Position und/oder Orientierung des medizinischen Instruments 1, d. h. die x-, y- und z-Koordinaten der Lokalisierungselemente 2 und 2' berechnet. Zum Zwecke der Kalibrierung ist im Koordinatenursprung ein Kalibrierungspunkt 10 vorgegeben. Zur Kalibrierung wird das Instrument 1 so positioniert und orientiert, dass sich dessen Spitze am Kalibrierungspunkt 10 befindet, wobei das Instrument 1 eine definierte Lage im Raum hat. Die mittels der Empfangseinheiten 6, 7 und 8 während der Kalibrierung detektierte Phasenlage des Lokalisierungssignals 5 bzw. 5' wird mittels der Auswertungseinheit 9 gespeichert. Bei der weiteren Positionsbestimmung setzt die Auswertungseinheit 9 die von den Empfangseinheiten 6, 7 und 8 empfangenen Signale zu den gespeicherten Kalibrierungsdaten in Beziehung, so dass die Positionen der Lokalisierungselements 2 und 2' relativ zum Koordinatenursprung bestimmt werden können.

Weiterhin ist in der Fig.1 ein zu dem System gehöriges zusätzliches Lokalisierungselement 2" dargestellt. Das Lokalisierungselement 2" ist nicht an dem medizinischen Instrument 1 angeordnet. Es ist mittels einer Klebeverbindung lösbar an der Hautoberfläche eines Patienten anbringbar. Der Transponder des zusätzlichen Lokalisierungselements 2" ist in einen selbstklebenden Gewebe- oder Folienstreifen, wie bei einem herkömmlichen Pflaster, integriert. Durch die von der Sendeeinheit 3 emittierte Strahlung 4 wird auch der Transponder des zusätzlichen Lokalisierungselements 2" angeregt, so dass dieser ein Lokalisierungssignal 5" aussendet. Anhand des Signals 5", das ebenfalls mittels der Detektionseinheiten 6,7 und 8 empfangen wird, ermittelt die Auswertungseinheit 9 die Position des zusätzlichen Lokalisierungselements 2". Dies ermöglicht es, die Position des Patienten sowie auch die Bewegungen des Patienten bei der Durchführung einer Intervention mittels des medizinischen Instrumentes 1 zu berücksichtigen.

In der folgenden Tabelle sind die Dämpfungswerte für die Signalübertragung zwischen Sendeeinheit 3, Lokalisierungselementen 2, 2', 2" und Empfangseinheiten 6, 7, 8 in Abhängigkeit von der Distanz d zwischen Sender und Empfänger für verschiedene typische RFID-Sendefrequenzen mit den dazugehörigen Wellenlängen zusammengestellt. Dabei wird auf Senderseite von einem Antennengewinn von 1,64 (Dipol) und auf Empfängerseite von einem Antennengewinn von 1,0 ausgegangen. In der Tabelle sind außerdem die Reichweiten bei den verschiedenen Frequenzen angegeben.

| Distanz *d* | 13,56 MHz | 433 MHz | 868 MHz (EU) | 915 MHz (US) | 2,45 GHz | 5,8 GHz |
|---|---|---|---|---|---|---|
| 0,3 m | - | 12,6 dB | 18,6 dB | 19,0 dB | 27,6 dB | 35,1 dB |
| 1 m | - | 23,0 dB | 29,0 dB | 29,5 dB | 38,0 dB | 45,5 dB |
| 2m | - | 29,0 dB | 35,1 dB | 35,5 dB | 44,1 dB | 51,6 dB |
| 3m | 2,4 dB | 32,6 dB | 38,6 dB | 39,0 dB | 47,6 dB | 55,1 dB |
| 10m | 12,9 dB | 43,0 dB | 49,0 dB | 49,5 dB | 58,0 dB | 65,6 dB |
| Reichweite: | 0-80 cm | 0-2 m | 0-5 m | 0-5 m | 0-100 m | 0-5 km |
| Wellenlänge: | 23 m | 69,2 cm | 34,5 cm | 32,5 cm | 12,24 cm | 5,17 cm |

Die Tabelle zeigt, dass sich die Anwendung des 433 MHz-Frequenzbereiches mit einem Arbeitsbereich von ca. 70 cm x 70 cm x 70 cm im Raum anbietet, wobei der Kalibrierungspunkt 10 maximal 2 m vom Sender bzw. Empfänger entfernt sein sollte. Wie zuvor beschrieben, erfolgt die Positionsbestimmung zweckmäßigerweise anhand der Phasenlage der Lokalisierungs-signale 5,5' und 5". Bei einer Frequenz von 433 MHz entspricht eine Phasendifferenz von 1° einem Abstand von 1,92 mm. Dementsprechend muss bei einer gewünschten räumlichen Auflösung von 1,92 mm die Auflösung bei der Bestimmung der Phasenlage wenigstens gleich 1° sein. Wird hingegen eine Frequenz von 5,8 GHz verwendet, so lässt sich bei einer Phasenauflösung von 1° bereits eine räumliche Auflösung von 0,14 mm erzielen. Zur Erzielung einer möglichst hohen Auflösung sind die Transponder der Lokalitierungselemente 2, 2' und 2" zweckmäßigerweise so eingerichtet, dass diese die Lokalisierungssignale 5, 5' und 5" bei zwei oder mehr verschiedenen Frequenzen erzeugen. Dadurch lässt sich eine Positionsbestimmung anhand der Phasenlage mit ausreichend hoher Genauigkeit erzielen. Durch die Verwendung von niedrigen Frequenzen kann die Position zunächst grob aber eindeutig bestimmt werden. Niedrige Frequenzen ergeben dabei einen vergleichsweise großen räumlichen Arbeitsbereich, innerhalb welchem die Positionsbestimmung erfolgen kann. Durch Übergang zu höheren Frequenzen kann dann die Position genauer bestimmt werden. Durch die Verwendung von mehreren Frequenzen ist eine eindeutige Bestimmung der Position anhand der Phasenlage mit gleichzeitig hoher Genauigkeit möglich.

Die Fig. 2 zeigt einen gemäß der Erfindung ausgestalteten intravaskulären Katheter 1. Der Katheter 1 wird mittels eines Führungsdrahts 11 innerhalb eines Blutgefäßes geführt. Der Katheter 1 ist mit einem Lokalisierungselement ausgestattet. Das Lokalisierungselement umfasst gemäß der Erfindung einen Transponder mit einem Schaltkreis 12 und einer Antenne 13. Die Antenne 13 ist aus dünnem Draht in Längsrichtung des Katheters 1 gewickelt und mit dem Schaltkreis 12 verbunden. Bei dem Schaltkreis 12 handelt es sich um einen integrierten Halbleiterchip. Mittels der Antenne 13 wird die von der Sendeeinheit 3 emittierte elektromagnetische Strahlung empfangen. Diese induziert in der Antenne 13 einen Induktionsstrom. Die Stromversorgung des Schaltkreises 12 erfolgt durch diesen Induktionsstrom. Bei dem in der Fig. 2 dargestellten Ausführungsbeispiel kommt also ein passiver Transponder zum Einsatz. Zur dauerhaften Energieversorgung des Schaltkreises 12 ist dieser mit einem Kondensator 14 verbunden, der durch den in der Antenne 13 erzeugten Induktionsstrom geladen wird. Der Kondensator 14 gewährleistet also die Funktion des Transponders, auch wenn der in der Antenne 13 erzeugte Induktionsstrom nicht kontinuierlich zur Energieversorgung ausreicht. Der Schaltkreis 12 wird durch die über die Antenne 13 empfangene elektromagnetische Strahlung aktiviert und dadurch zur Abstrahlung eines Lokalisierungssignals als elektromagnetische Strahlung über die Antenne 13 angeregt. Dies erfolgt, indem der Schaltkreis 12 eine Lastmodulation des mittels der Antenne 13 empfangenen elektromagnetischen Feldes bewirkt. Der Schaltkreis 12 ist des Weiteren mit einem in den Katheter 1 integrierten Sensorelement 15, beispielsweise einem Temperatursensor verbunden. Der Schaltkreis 12 des Transponders überträgt das Sensorsignal des Sensor-elements 15 als Digitalsignal über die Antenne 13. Dies ermöglicht es, drahtlos die Temperatur am jeweiligen Ort der Spitze des Katheters 1 zu bestimmen.

## Patentansprüche

1. System zur Bestimmung der räumlichen Position und/oder Orientierung eines medizinischen Instrumentes (1), mit einer elektromagnetische Strahlung (4) emittierenden Sendeeinheit (3), wenigstens einem an dem medizinischen Instrument (1) angeordneten Lokalisierungselement (2), welches geeignet ist, die von der Sendeeinheit (3) emittierte elektromagnetische Strahlung (4) zu empfangen und ein Lokalisierungssignal (5) zu erzeugen, einer Auswertungseinheit (9), welche eingerichtet ist, die Posibon und/oder Orientierung des medizinischen Instrumentes (1) durch Auswertung des Lokalisierungssignals (5) zu bestimmen, und mit wenigstens einer mit der Auswertungseinheit (9) verbundenen Empfangseinheit (6, 7, 8), wobei das Lokalisierungselement (2) einen Transponder aufweist, der eine Antenne (13) und einen mit der Antenne (13) verbundenen Schaltkreis (12) zum Empfangen und Sendern von elektromagnetischer Strahlung umfasst, wobei der Schaltkreis (12) durch die über die Antenne empfangene elektromagnetische Strahlung (4) der Sendeeinheit (3) anregbar ist, und zwar in der Weise, dass dieser das Lokalisierungssignal (5) als elektromagnetische Strahlung über die Antenne (13) aussendet,
**dadurch gekennzeichnet, dass** die Auswertungseinheit (9) zur Bestimmung der Position und/oder Orientierung des medizinischen Instrumentes (1) anhand der Phasenlage der elektromagnetischen Strahlung des Lokalisierungssignals (5) am jeweiligen Ort der Empfangseinheit (6, 7, 8) eingerichtet ist, wobei der Schaltkreis (12) zur Erzeugung des Lokalisierungssignals bei zwei oder mehr verschiedenen Frequenzen eingerichtet ist, in der Weise, dass durch die Erzeugung des Lokalisierungssignals bei einer niedrigeren Frequenz eine grobe Bestimmung der Position und/oder Orientierung erfolgt und zur Steigerung der Genauigkeit zu einer höheren Frequenz übergegangen wird oder die Frequenz des Lokalisierungssignals sukzessive gesteigert wird.

2. System nach Anspruch 1, **dadurch gekennzeichnet, dass** sich die Frequenz der elektromagnetischen Strahlung des Lokalisierungssignals (5) von der Frequenz der von der Sendeeinheit (3) emittierten elektromagnetischen Strahlung (4) unterscheidet.

3. System nach Anspruch 1 oder 2, dadurch gekenntzeichnet, dass der Transponder ein RFID-Tag ist.

4. System nach einem der Ansprüche 1 bis 3, **dadurch gekennzeichnet, dass** an dem medizinischen Instrument (1) wenigstens zwei Lokalisierungselemente (2, 2') mit diesen jeweils zugeordneten Transpondern angeordnet sind.

5. System nach einem der Ansprüche 1 bis 4, **gekennzeichnet durch** wenigstens ein nicht an dem medizinischen Instrument (1) angeordnetes, zusätzliches Lokalisierungselement (2") mit einem diesem zugeordneten Transponder, welches am Körper eines Patienten lösbar anbringbar ist.

6. System nach einem der Ansprüche 1 bis 5, **dadurch gekennzeichnet, dass** die Sendeeinheit (3) die Sendeeinheit eines MR-Gerätes ist, welche eine Sende-/Empfangsantenne (Spule) zur Erzeugung eines hochfrequenten elektromagnetischen Feldes im Üntersuchungsvolumen des MR-Gerätes umfasst.

7. System nach Anspruch 6, **dadurch gekennzeichnet, dass** der Transponder als passiver Transponder ausgebildet ist, wobei die Stromversorgung des Schaltkreises (12) durch den beim Empfang des hochfrequenten elektromagnetischen Feldes während der MR-Bildgebung in der Antenne (13) erzeugten Induktionsstrom erfolgt.

8. System nach Anspruch 6 oder 7, **dadurch gekennzeichnet, dass** die Auswertungseinheit (9) mit dem MR-Gerät verbunden ist, wobei die Bestimmung der Position und/oder Orientierung des medizinischen Instrumentes (1) anhand des über die Sende-/Empfangsantenne (Spule) des MR-Gerätes empfangenen Lokalisierungssignals (5) erfolgt.

9. System nach einem der Ansprüche 1 bis 8, **dadurch gekennzeichnet, dass** die Auswertungseinheit (9) zur Auswahl gültiger Positions- und/oder Orientierungsdaten aus einer Mehrzahl von redundant aus mehreren Lokalisierungssignalen (5) bestimmten Position- und/oder Orientierungsdaten eingerichtet ist.

10. System nach Anspruch 9, **dadurch gekennzeichnet, dass** an dem medizinischen Instrument (1) zur Erzeugung redundanter Lokalisierungssignale (5) mehrere Lokalisierungselemente (2) angeordnet sind.

11. Verfahren zur Bestimmung der räumlichen Position und/oder Orientierung eines medizinischen Instrumentes (1), wobei mittels einer Sendeeinheit (3) elektromagnetische Strahlung (4) emittiert wird, die von wenigstens einem an dem medizinischen Instrument (1) angeordneten Lokalisierungselement (2) empfangen wird, woraufhin das Lokalisierungselement (2) ein Lokalisierungssignal (5) erzeugt, und wobei mittels einer Auswertungseinheit (9) die Position und/oder Orientierung des medizinischen Instrumentes (1) durch Auswertung des Lokalisierungssignals (5) bestimmt wird, wobei das Lokalisierungselement (2) einen Transponder aufweist, der eine Antenne (13) und einen mit der Antenne (13) verbundenen Schaltkreis (12) zum Empfangen und Senden von elektromagnetischer Strahlung umfasst, wobei der Schaltkreis (12) durch die über die Antenne empfangene elektromagnetische Strahlung (4) der Sendeeinheit (3) angeregt wird, woraufhin dieser das Lokalisierungssignal (5) als elektromagnetische Strahlung über die Antenne (13) aussendet,
**dadurch gekennzeichnet, dass** die Position und/oder Orientierung des medizinischen Instrumentes (1) anhand der Phasenlage der elektromagnetischen Strahlung des Lokalisierungssignals (5) am Ort wenigstens einer mit der Auswertungseinheit (9) verbundenen Empfangseinheit (6, 7, 8) bestimmt wird, wobei das Lokalisierungssignal bei zwei oder mehr verschiedenen Frequenzen erzeugt wird, in der Weise, dass durch die Erzeugung des Lokalisierungssignals bei einer niedrigeren Frequenz eine grobe Bestimmung der Position und/oder Orientierung erfolgt und zur Steigerung der Genauigkeit zu einer höheren Frequenz übergegangen wird oder die Frequenz des Lokalisierungssignals sukzessive gesteigert wird.

12. Verfahren nach Anspruch 11, **dadurch gekennzeichnet, dass** das medizinische Instrument (1) ein intravaskulärer Katheter, ein Führungsdraht oder eine Biopsienadel ist.

13. Verfahren nach Anspruch 11 oder 12, **dadurch gekennzeichnet, dass** der Transponder ein RFID-Tag ist.

## Claims

1. A system for determining the spatial position and/or orientation of a medical instrument (1), comprised of a transmission unit (3) emitting an electromagnetic radiation (4), at least one localisation element (2) arranged at a medical instrument (1) which is suitable for receiving the electromagnetic radiation (4) emitted from transmission unit (3) and for generating a localisation signal (5), and comprised of an evaluation unit (9), which is configured to determine the position and/or orientation of the medical instrument (1) by evaluating the localisation signal (5), and at least one receiving unit (6, 7, 8) connected to the evaluation unit (9), wherein the localisation element (2) is comprised of a transponder which is comprised of an antenna (13) and a circuit (12) connected to the antenna (13) for receiving and transmitting electromagnetic radiation, with said circuit (12) being excitable through the electromagnetic radiation (4) from the transmission unit (3) received via the antenna, in such a manner that it emits the localisation signal (5) as electromagnetic radiation through antenna (13),
**characterized in that** the evaluation unit (9) being properly provided for determining the position and/or orientation of the medical instrument (1) based on the phase relation of the electromagnetic radiation of the localisation signal (5) at the relevant site of the receiving unit (6, 7, 8), wherein the circuit (12) is provided for generating the localisation signal at two or more different frequencies, in such a way, that by generating the localisation signal at low frequencies a rough determination of the position and/or orientation takes place, and to increase accuracy a higher frequency is then chosen or the frequency of the localisation signal is successively incremented.

2. A system as defined in claim 1, **characterized in that** the frequency of the electromagnetic radiation of the localisation signal (5) differs from the frequency of the electromagnetic radiation (4) emitted from the transmission unit (3).

3. A system as defined in claim 1 or 2, **characterized in that** the transponder is an RFID tag.

4. A system as defined in any of the preceding claims 1 to 3, **characterized in that** at the medical instrument (1) at least two localisation elements (2, 2') with two transponders allocated to them are arranged.

5. A system as defined in any of the preceding claims 1 to 4, **characterized by** at least one additional localisation element (2") not arranged at the medical instrument (1) with a transponder allocated to it which can be affixed in detachable arrangement at a patient's body.

6. A system as defined in any of the preceding claims 1 to 5, **characterized in that** the transmission unit (3) is the transmission unit of an MR device which is comprised of a transmission/receiving antenna (coil) to generate a high-frequency electromagnetic field in the investigation volume of the MR device.

7. A system as defined in claim 6, **characterized in that** the transponder is configured as a passive transponder, with the power supply to the circuit (12) being provided by the induction current generated in the antenna (13) on reception of the high-frequency electromagnetic field during the MR imaging.

8. A system as defined in claim 6 to 7, **characterized in that** the evaluation unit (9) is linked to the MR device, with the determination of the position and/or orientation of the medical instrument (1) being effected based on the localisation signal (5) received via the transmission/receiving antenna (coil) of the MR device.

9. A system as defined in any of the preceding claims 1 to 8, **characterized in that** the evaluation unit (9) is provided for selecting valid position and/or orientation data from a plurality of position and/or orientation data redundantly determined from several localisation signals (5).

10. A system as defined in claim 9, **characterized in that** several localisation elements (2) are arranged at the medical instrument (1) to generate redundant localisation signals (5).

11. A method for determining the spatial position and/or orientation of a medical instrument (1), wherein electromagnetic radiation (4) is emitted by means of a transmission unit (3) which is received by at least one localisation element (2) arranged at the medical instrument (1), whereupon the localisation element (2) generates a localisation signal (5) and wherein by means of an evaluation unit (9) the position and/or orientation of the medical instrument (1) is determined by evaluating the localisation signal (5), wherein the localisation element (2) is comprised of a transponder which is comprised of an antenna (13) and a circuit (12) connected to the antenna (13) for receiving and transmitting electromagnetic radiation, with said circuit (12) being excited by the electromagnetic radiation (4) from the transmission unit (3) received via the antenna, whereupon it emits the localisation signal (5) as electromagnetic radiation via the antenna (13)
**characterized in that** the position and/or orientation of the medical instrument (1) is determined based on the phase relation of the electromagnetic radiation of the localisation signal (5) at the site of at least one receiving unit (6, 7, 8) connected to the evaluation unit (9), wherein the localisation signal is generated at two or more different frequencies, in such a way, that by generating the localisation signal at low frequencies a rough determination of the position and/or orientation takes place, and to increase accuracy a higher frequency is then chosen or the frequency of the localisation signal is successively incremented..

12. A method as defined in claim 11, **characterized in that** the medical instrument (1) is an intravascular catheter, a guidance wire or a biopsy needle.

13. A method as defined in claim 11 or 12, **characterized in that** the transponder is an RFID tag.

## Revendications

1. Système de détermination de la position et/ou de l'orientation d'un instrument médical (1), comprenant une unité d'émission (3) d'un rayonnement électromagnétique (4), au moins un élément de localisation (2) disposé sur l'instrument médical (1), qui est adapté pour recevoir le rayonnement électromagnétique (4) émis par l'unité d'émission (3) et engendrer un signal de localisation (5), une unité d'exploitation (9) qui est adaptée pour déterminer la position et/ou l'orientation de l'instrument médical (1) par exploitation du signal de localisation (5), et avec au moins une unité de réception (6, 7, 8) relié à l'unité d'exploitation (9), l'élément de localisation (2) comportant un transpondeur qui comprend une antenne (13) et un circuit (12) relié à l'antenne (13) pour recevoir et émettre un rayonnement électromagnétique (4), le circuit (12) étant excitable par le rayonnement électromagnétique (4) de l'unité d'émission (3) reçue par l'antenne, et cela de la manière qu'il émet le signal de localisation (5) par l'antenne (13) comme rayonnement électromagnétique,
**caractérisé en ce que** l'unité d'exploitation (9) est adapté pour la détermination de la position et/ou de l'orientation de l'instrument médical (1) sur la base de la position de phase du rayonnement électromagnétique du signal de localisation (5) à l'endroit respectif de l'unité de réception (6, 7, 8), le circuit (12) étant adapté pour engendrer le signal de localisation à deux ou plus de fréquences différentes, de la manière que par la génération du signal de localisation à une fréquence plutôt basse, la position et/ou l'orientation sont déterminées grossièrement, et pour augmenter la précision, on utilise une fréquence plus élevée ou la fréquence du signal de localisation est augmentée successivement.

2. Système selon la revendication 1, **caractérisé en ce que** la fréquence du rayonnement électromagnétique du signal de localisation (5) diffère de la fréquence du rayonnement électromagnétique (4) émis par l'unité d'émission (3).

3. Système selon la revendication 1 ou 2, **caractérisé en ce que** le transpondeur est une balise RFID.

4. Système selon l'une des revendications 1 à 3, **caractérisé en ce qu'**au moins deux éléments de localisation (2, 2') et des transpondeurs respectivement associés sont disposés sur l'instrument médical (1).

5. Système selon l'une quelconque des revendications 1 à 4, **caractérisé par** un élément de localisation (2'') supplémentaire avec un transpondeur associé, qui n'est pas disposé sur l'instrument médical (1) et qui peut être disposé de manière amovible sur le corps d'un patient.

6. Système selon l'une quelconque des revendications 1 à 5, **caractérisé en ce que** l'unité d'émission (3) est l'unité d'émission d'un appareil RM, laquelle comprend une antenne d'émission et de réception (bobine) pour engendrer un champ électromagnétique de haute fréquence dans le volume d'exploration de l'appareil RM.

7. Système selon la revendication 6, **caractérisé en ce que** le transpondeur est agencé comme transpondeur passif, l'alimentation en courant du circuit (12) étant effectuée par le courant d'induction engendré par l'antenne (13) par la réception du champ électromagnétique à haute fréquence pendant la génération d'image RM.

8. Système selon la revendication 6 ou 7, **caractérisé en ce que** l'unité d'exploitation (9) est relié à l'appareil RM, la détermination de la position et/ou de l'orientation de l'instrument médical (1) étant effectuée sur la base du signal de localisation (5) reçu par l'antenne d'émission et de réception (bobine) de l'appareil RM.

9. Système selon l'une quelconque des revendications 1 à 8, **caractérisé en ce que** l'unité d'exploitation (9) est adaptée pour sélectionner des données valides de position et/ou d'orientation parmi une pluralité de données de position et/ou d'orientation déterminées de manière redondante à partir de plusieurs signaux de localisation (5).

10. Système selon la revendication 9, **caractérisé en ce que** plusieurs éléments de localisation (2) sont disposés sur l'instrument médical (1) pour engendrer des signaux de localisation (5) redondants.

11. Procédé de détermination de la position et/ou de l'orientation d'un instrument médical (1), selon lequel un rayonnement électromagnétique (4) est émis par une unité d'émission (3), qui est reçu par un élément de localisation (2) disposé sur l'instrument médical (1), l'élément de localisation (2) engendrant un signal de localisation (5), et la position et/ou l'orientation de l'instrument médical (1) étant déterminées par une unité d'exploitation (9) par exploitation du signal de localisation (5), l'élément de localisation (2) comportant un transpondeur comportant une antenne (13) et un circuit (12) relié à l'antenne (13) pour recevoir et émettre du rayonnement électromagnétique, le circuit (12) étant excité par le rayonnement électromagnétique (4) de l'unité d'émission (3) reçue par l'antenne, et cela de la manière qu'il émet le signal de localisation (5) par l'antenne (13) comme rayonnement électromagnétique,
**caractérisé en ce que** la position et/ou de l'orientation de l'instrument médical (1) sont déterminées sur la base de la position de phase du rayonnement électromagnétique du signal de localisation (5) à l'endroit respectif d'au moins une unité de réception (6, 7, 8) reliée à l'unité d'exploitation (9), le signal de localisation étant engendré à deux ou plus de fréquences différentes, de la manière que par la génération du signal de localisation à une fréquence plutôt basse, la position et/ou l'orientation sont déterminées grossièrement, et pour augmenter la précision, on utilise une fréquence plus élevée ou la fréquence du signal de localisation est augmentée successivement.

12. Système selon la revendication 11, **caractérisé en ce que** l'instrument médical (1) est un cathéter intravasculaire, un fil de guidage ou une aiguille de biopsie.

13. Système selon la revendication 11 ou 12, **caractérisé en ce que** le transpondeur est une balise RFID.
